# NEUE EUROPÄISCHE PATENTSCHRIFT

(11) **EP 1 577 269 B2**
(45) Veröffentlichungstag und Bekanntmachung des Hinweises auf die Entscheidung über den Einspruch: **17.12.2014**
(45) Hinweis auf die Patenterteilung: 11.07.2007
(21) Anmeldenummer: 05450052.5
(22) Anmeldetag: 17.03.2005
(51) Int. Cl.: C02F 11/04, C02F 3/28

(54) **Verwendung von mehrfach modifiziertem Zeolith in der Biogasgewinnung**
Use of polymodified Zeolite in Biogas winning
Utilisation d'une zéolithe polymodifiée dans l'obtention du biogaz

(30) Priorität: 17.03.2004 AT 4642004
(43) Veröffentlichungstag der Anmeldung: 21.09.2005
(73) Patentinhaber: IPUS Industrie-Produktions- und Umwelttechnisches Service GmbH, 8786 Rottenmann (AT)
(72) Erfinder: Holper, Josef, 8053 Graz (AT); Lesjak, Meinhard, 8943 Aigen im Ennstal (AT); Heinzel, Ute, 76597 Loffenau (DE); Boos, Bernd, 76332 Bad-Herrenalb (DE)
(74) Vertreter: Burger, Hannes

(56) Entgegenhaltungen:
- RU-C1- 2 096 348
- Z. MILAN, P. VILLA, E. SANCHEZ, S. MONTALVO, R. BORJA, K. ILANGOVAN AND R. BRIONES: "Effect of natural and modified zeolite addition on anaerobic digestion of piggery waste" WATER SCIENCE AND TECHNOLOGY, Bd. 48, Nr. 6, 2003, Seiten 263-269, XP002331116
- C. DE LAS POZAS ET AL.: 'Location and Reducibility of Ni Ions in HEU-Type Zeolites' JOURNAL OF SOLID STATE CHEMISTRY Bd. 114, 1995, Seiten 108 - 111

## Beschreibung

Die Erfindung betrifft die Verwendung von Zeolith zur Gewinnung von Methangas, entsprechend dem Oberbegriff des Anspruchs 1.

Es ist bekannt, Zeolith in Kläranlagen zu verwenden, wo vor allem aerobe bakterielle Prozesse zum Abbau der Schadstoffe im Eintrag stattfinden. Der Eintrag weist in der Regel einen geringeren Anteil an Trockensubstanz von organischem Material mit relativ niedrigem Anteil an Schadstoffen auf. Zeolith nimmt diese - für die Bakterien nahrhaften Stoffe - auf und stellt sie für die Bakterien "greifbarer" zur Verfügung. Die Vermehrung der Bakterienpopulationen wird gesteigert und damit einhergehend die Reduzierung des Schadstoffanteils.

In der EP 0 173 340 A2 ist ein Verfahren zur Kultivierung von Mikroorganismen im wässrigen Milieu, insbesondere in einem Sandfilter, beschrieben, wobei der Sandfilter zur Klärung von Schlämmen verwendet wird. Zur Steigerung der Wachstumsrate wird dem Nährmedium natürlicher oder synthetischer Zeolith beigemengt.

Die US 3 224 946 A beschreibt ein Verfahren zur Steigerung der mikrobiellen Fermentationsrate mit Hilfe von Zeolith als Träger, wobei die im Substrat vorhandenen Kohlenwasserstoffe abgebaut werden.

Diese Verfahren gemäß dem Stand der Technik arbeiten im wässrigen Medium.

Die Gewinnung von Methangas oder Biogas zur Energieerzeugung gewinnt immer mehr an Bedeutung. Als Gärsubstrat werden feste und flüssige biogene Abfallstoffe in der Regel aus der Industrie, aus dem Gewerbe, aus der Landwirtschaft, aus Haushalten und aus Abwasserreinigungsanlagen eingesetzt. Die Biogasgewinnung erfolgt anaerob in einem Fermenter unter kontrollierten Bedingungen bei etwa 30°C bis 40°C oder bei etwa 50°C bis 60°C und basiert auf der Vergärung oder - bei Abfall aus der Landwirtschaft bevorzugte - Co-Vergärung.

Das Gärsubstrat weist einen relativ hohen Anteil an organischer Substanz von organischem Material und einen relativ hohen Anteil an Schadstoffen auf. Das Gärsubstrat wird mithilfe von Methanbakterien stufenweise, in im Wesentlichen vier Phasen, unter Bildung von im Wesentlichen Methan, Kohlendioxid und Wasser abgebaut (etwa 2/3 Methan, etwa 1/3 Kohlendioxid und Restgase). In Abhängigkeit der Bakterien ist der Gärungsprozess mesophil (etwa bei 35°C) oder thermophil (etwa bei 55°C). Die typischerweise am Gärungsprozess teilhabenden Bakterien sind beispielsweise Acidomonas methanol., Acetobacterium malicum, Acetobacterium carbinolicum, Methanobacterium thermoalcaliphil., Clostidium ultense, Methanobacterium sp., Methanospirillium hungatei, Sporotomaculum syntrophicum, Methanosaeta concilii, Methanothrix concilii, Methanoococcus mazei, Syntrophomonas sapovorans, etc. Sie werden der Biomasse im Fermenter - je nach Art des Gärsubstrats - als sogenanntes Impfmaterial zugegeben.

Eine zufriedenstellende Vergärung wird bei einem pH-Wert von im Wesentlichen zwischen 6,8 bis 8,5, am günstigsten im Bereich von 7,5 erreicht. Von Wichtigkeit ist das Nährstoffverhältnis, nämlich das Verhältnis (CNP-Verhältnis) von Kohlenstoff, Stickstoff, Phosphor, aber auch Spurenelementen. Das CNP-Verhältnis ist günstigerweise etwa bei C:N 10 bis 16:1 und N:P 7:1 zu halten.

Durch vielfältige Störungen im biologischen Prozessablauf können sich die Methangasbakterien nicht in gewünschter Weise vermehren, wodurch die Reaktionen zur Bildung des Methans nur gehemmt bzw. verzögernd ablaufen können. Ursache hiefür sind vermehrt Stoffe, wie beispielsweise Ammoniumstickstoff, insbesondere in der Form NH₄⁺ oder NH₃, Schwefelverbindungen und/oder Schwermetallkonzentrationen, die, wenn im Übermaß im Gärsubstrat vorhanden, auf die Methanbakterien toxisch wirken und dadurch die ihre Bioaktivität hemmen oder gar verhindern.

Oftmals wird eine Schaumbildung im Fermenter beobachtet, die durch einen Eiweißüberschuss im Gärsubstrat ausgelöst wird. Der pH-Wert fällt in den sauren Bereich und es folgt ein Anstieg an undissoziierten Säuren, wie z.B. Essigsäure, Buttersäure, Propionsäure, Valeriansäure, etc. Die Gasproduktion sinkt ab, der Gehalt an Kohlendioxid steigt und es kommt zur Schaumbildung.

Nicht nur das Ammonium sondern auch eine erhöhte Zahl an Schwefelverbindungen im Gärsubstrat zeigt eine hemmende Wirkung auf die Aktivität der Methanbakterien. In Abhängigkeit vom pH-Wert werden Sulfide bzw. Schwefelwasserstoff gebildet. Insbesondere hat der Schwefelwasserstoff eine toxische Wirkung, vor allem in der flüssigen Phase. Die Anwesenheit von Schwefelwasserstoff ist auch in bezug auf die Verwertung des gewonnen Methangases - im Zuge seiner thermischen Umsetzung - aufgrund der Bildung von Schwefelsäure (Korrosionsschäden) nachteilig.

Um diese Probleme zu vermeiden und das Gefährdungspotential nachteilig.

Um diese Probleme as vermeiden und das Gefährdungspotential für die Methanbakterien zu verringern, wurde durch Zugaben von Tonmineralien, insbesondere Bentonit, versucht, die toxischen Stoffe zu immobilisieren. Allerdings konnten damit nur bedingt gute Erfolge erzielt werden. Eine wesentliche Erhöhung der Gasausbeute in einer kürzeren Faulzeit konnte nicht beobachtet werden.

Ebenso ist bekannt, dem Absinken des pH-Wertes durch Zusatz von Kalkmilch, Natronlauge, etc. entgegenzutreten. Zur Verhinderung der Schwefelwasserstoffbildung werden oft Eisensalze, insbesondere Eisenchlorid beigemengt. Gleichfalls ist die Zugabe von Enzympräparaten auf Basis von Amylase, Cellulase und dergleichen zwecks Beschleunigung der Umsetzung der Biomasse bekannt. Diese Maßnahmen führen allerdings nicht zur Behebung der Ursache der Störungen in den anearoben Abbauprozessen.

Die JP 60 014 995 A beschreibt ein Verfahren zur Verbesserung der Fermentation bei der Erzeugung von Methangas. Hiefür werden dem Fermentationsprozess natürliche Feststoffe beigemengt, wobei neben zahlreichen Silikatmineralien auch das Mineral Zeolith verwendet wird. Selbst mit diesem vorgeschlagenen Verfahren konnten die vorerwähnten Schwierigkeiten nur ungenügend beseitigt werden.

In "Effect of natural and modified zeolite addition on anaerobic digestion of piggery waste" (Z. Milán et al., Water Science and Technology, Vol 48 Nr. 6, Seiten 263-269) wird der Effekt von natürlichen oder modifizierten Zeolithen in Hinblick auf den anaeroben Abbau von Acetat und Methanol beschrieben. Der Zeolith ist in einer Menge von 0,01 g bzw. 0,05 g bzw. 0,1 g/g VSS (volatile suspended solids) zugesetzt. Es wird ein natürlicher sowie mit einem einfach modifizierten Zeolith verglichen, wobei als Gärsubstrat Abfall aus der Schweinezucht verwendet wird. Der Zeolith wird mit Magnesium, Kobalt oder Nickel modifiziert, wobei hinsichtlich der Methanproduktion die größte Beschleunigung des Abbauprozesses mit Magnesium und die kleinste mit Nickel beobachtet wurde.

Es ist nun Aufgabe der vorliegenden Erfindung die vorstehend genannten Probleme zu vermeiden und die Ursache des Gefährdungspotentials für die Methanbakterien möglichst auszuschalten, um die Bedingungen für die Methangasgewinnung zu optimieren, sodass die Gasausbeute gesteigert und die Faulzeit verkürzt werden kann.

Dies wird erfindungsgemäß durch die Merkmale in Kennzeichenteil des Anspruchs 1 gelöst.

Unter einem modifizierten Zeolith (nachstehend auch als aktivierter Zeolith bezeichnet) wird gemäß der Erfindung ein chemisch behandelter Zeolith verstanden der Cobalt- und Eisen aktiviert wurde, wobei nach Modifizierung ein veränderter zeolith vorliegt. Unter chemisch, behandelter zeolith fällt gemäß der Erfindung ein beladener, versetzter Zeolith. Dementsprechend weist ein modifizierter Zeolith Fremdionen auf.

Eine chemische Modifizierung des Zeoliths kann beispielsweise dadurch erhalten werden, dass pulverisierter Zeolith von einer Lösung infiltriert wird, die die Aktivierungssubstanz enthält. Die Aktivierungssubstanz wird dann durch chemische Bindung an den Zeolith gebunden.

Gemäß der Erfindung wird die organische Substanz durch die an sich bekannte Trockensubstanz-Bestimmung bei 120°C und anschließende Glühverlust-Bestimmung bei 400°C erhalten oder durch die ebenfalls an sich bekannte Messung des chemischen Sauerstoffbedarfs (CSB) ermittelt.

Durch erfindungsgemäße die Verwendung von mehrfach modifiziertem Zeolith (nachstehend auch nur als Zeolith bezeichnet) kann im Gärsubstrat den vorstehend genannten Störungen entgegengewirkt werden. Der Zeolith kann als katalytisch wirkendes Additiv den Gärungsprozess beschleunigen, wobei er sowohl die chemischen als auch die biologischen Prozesse in geeigneter Weise anregen kann. Dadurch kann das Gärverhalten verbessert, die Methangasproduktion beschleunigt und ein hoch qualitatives Biogas geliefert werden. Gleichfalls kann der Gärrückstand für die nachfolgende Verarbeitung, beispielsweise für die Herstellung eines Düngers, verbesserte Eigenschaften aufweisen, wie eine höhere Wasserspeicherkapazität, Ammoniumanreicherung, Kaliumanreicherung.

Die erfindungsgemäße Verwendung von modifiziertem Zeolith richtet sich nach der Art des Gärsubstrat, die jahreszeitlich bedingt ist, wobei sich jeweils sehr unterschiedliche Ausgangsbedingungen für die Methanbakterien einstellen, wie Eiweißüberschuss oder -mangel im Gärsubstrat, Mangel an Spurenelementen, Überschuss an Schwefelverbindungen, etc.

Durch die vielfältige Wirkungsweise des Zeoliths können die unterschiedlichen Ausgangsbedingungen im Gärsubstrat für die Methanbildung ausgeglichen werden. Dies führt zu einer Milieuverbesserung und optimierten Zellteilung im Gärsubstrat. Die vorgenannte ammoniakinduzierte Hemmung der Gärung kann verringert und die Schwefelwasserstoffkonzentrationen im Biogas können reduziert werden.

Die erfindungsgemäße Anwendung von Zeolith kann eine Erhöhung der Bakterienpopulationen und damit einhergehend eine Steigerung der Enzymproduktion herbeiführen. Dadurch können die Hydrolyseprodukte in kürzerer Zeit gebildet werden. Die Vergärung setzt rascher ein, wodurch die Gärungsprozessdauer verringert und die Gasproduktion erhöht werden kann.

Der Zeolith kann in einem eiweißhältigen Gärsubstrat nicht nur immobilisierend in Bezug auf das für die Methanbakterien toxische Ammonium wirken, sondern auch hinsichtlich der Schwefelverbindungen und Schwermetalle. Gleichfalls können andere hemmende organischen Verbindungen, wie PCB (polychlorierte Biphenyle), AOX (halogenierte Kohlenwasserstoffverbindungen), THM (Trihalogenmethane) und dergleichen gebunden werden. Dies beruht auf der durch die Modifizierung des Zeoliths verstärkten Ionenaustauschkapazität und Sorptionsfähigkeit. Er reagiert anionisch und kann daher als Ionenaustauscher gegenüber Kationen verstärkt genutzt werden. Aufgrund seiner Kristallstruktur, ein Alumosilikat-Gerüst, welche durch die räumliche Anordnung der SiO₄- und AlO₄-Tetraeder charakterisiert ist, weist er Hohlräume auf, die als Adsorptionsfläche dienen kann. Durch die relativ große innere und obere Oberfläche können nicht nur Feststoffe und Flüssigkeiten, sondern auch Gase adsorbiert werden. Die toxischen Stoffe Ammoniak und Schwefelwasserstoff können gebunden, sorbiert bzw. dissoziiert werden, und liegen somit in einer für die Methangasbakterien unschädlichen Form im Gärsubstrat vor. Zeolith kann durch seine Ionenaustauschkapazität die schwankenden pH-Werte, beispielsweise dem Absinken in den sauren Bereich, puffern und die Abbauprozesse im Fermenter stabilisieren. Die fallweise auftretende Schaumentwicklung im Fermenter, wobei als Gärsubstrat beispielsweise Klärschlamm aus der Belebung und vorklärung (Primär- und Sekundärschlamm) von Abwasserreinigungsanlagen eingesetzt wird, kann durch die Anwesenheit von Zeolith im Gärsubstrat verhindert werden. Gleichfalls kommt die nachgewiesene verstärkte katalytische Wirkung des Zeoliths dem mikrobiologischen Abbau des Gärsubstrates zugute. Am Zeolith können aber auch für die Mikroorganismen wichtige Spurenelmente, auch Schwermetalle, in der wässrigen Phase dissoziiert werden und den Mikroorganismen für dessen Zellteilung zur Verfügung stehen.

Ein weiterer Vorteil des modifizierten Zeoliths ist, dass die Geruchsbildung während der Gärung und aus dem Gärrückstand reduziert werden kann.

Es hat sich gezeigt, dass die Menge von etwa 0,1% bis zu etwa 10% Zeolith bezogen auf die organische Substanz des Gärsubstrats sich besonders milieuverbessernd und sich somit günstig auf die Steigerungsrate der Methanbakterienpopulationen auswirkt. Je nachdem, ob der Gärungsprozess diskontinuierlich, d.h. batchweise, semikontinuierlich und bzw. oder kontinuierlich im Fermenter abläuft, kann die Zugabe von Zeolith an die Vorgehensweisen adaptiert werden. Im Falle der diskontinuierlichen Vergärung kann beispielsweise derart vorgegangen werden, dass der Zeolith dem Gärsubstrat einmalig beigemengt wird. Bei kontinuierlicher Verfahrensweise kann eine chargenweise Zudosierung des Zeoliths vorteilhaft sein.

In der Regel kann das zeolithische Material nach Abschluss des Gärungsprozesses im Gärrückstand verbleiben. Dies ist sogar hinsichtlich der Weiterbehandlung des Gärrückstandes vorteilhaft, welche gängigerweise in der Verrottung und der anschließenden Kompostierung des Gärrückstandes besteht. Da im Zuge der Vergärung mehr organisches Material aufgrund der Anwesenheit von Zeolith abgebaut werden kann, weist der Gärrückstand einen höheren Anteil an mineralischen Komponenten und demnach einen geringeren Wassergehalt auf. Ein geringerer Wassergehalt im Gärrückstand begünstigt eine raschere Verrottung sowie in weiterer Folge eine bessere Kompostierung, wodurch ein hinsichtlich der Nährstoffe qualitativ höheres Endprodukt erhalten werden kann.

Zur Modifizierung, d.h. als Ausgangsprodukt des modifizierten Zeoliths, kann ein natürlicher Zeolith verwendet werden. Dabei ist es denkbar, einen natürlichen Zeolith einer Kombination aus chemisch und chemisch-physikalischen Behandlungen zu unterziehen, wobei die Behandlungen gleichzeitig vorgenommen werden können, um einen sozusagen doppelt modifizierten Zeolith zu erhalten und so als Zusatz für das Gärsubstrat zu verwenden.

Es sei angemerkt, dass unter natürlichem Zeolith im allgemeinen unbehandelter Zeolith mit im Wesentlichen der Summenformel Na, Ca, K, Na (Al₂Si₃₀O₇₂) 24 H₂O verstanden wird.

Bei der Wahl eines natürlichen Zeoliths können aufgrund der Gestalt ihrer Kristalle bevorzugterweise Klinoptilolith, Chabasit, Phillipsit, Analcim oder ähnliche, sowie Mischungen hievon, zum Einsatz kommen.

Zahlreiche, insbesondere natürliche Zeolithe, sind von Quarzkristallen begleitet. Nach einer mechanischen Aufbereitung des zeolithischen Materials auf die gewünschte Korngröße liegt der Quarz lose im Material vor. Bei der Verwendung eines quarzhältigen zeolithischen Materials als Ausgangsprodukt kann es allerdings leicht zu Abrasionen im Fermenter, insbesondere der Rohrleitungen, Pumpen, Messsonden, etc. kommen. Um dies zu vermeiden ist es vorteilhaft, Zeolithe mit einem geringeren Anteil an Quarzkristallen einzusetzen. Am bevorzugtesten sind quarzfreie Zeolithe.

In jedem Fall sind Zeolith-Kristalle vorteilhaft, die eine gedrungene, tafelige, prismatische und ähnliche Gestalt aufweisen. Es hat sich gezeigt, dass die Verwendung von faserigen bzw. nadeligen Zeolith-Kristallen aufgrund des höheren Gesundheitsrisikos zu vermeiden ist.

Gemäß der Erfindung wird ein Zeolith gemäß den Ansprüchen als Zusatz im Gärsubstrat verwendet werden. Es sei angemerkt, dass für einen zwei- oder mehrfach modifizierten Zeolith nicht die gleichen Behandlungen gewählt werden müssen. Auf diese Weise können Eigenschaften unterschiedlich modifizierter Zeolithe miteinander kombiniert und in einem Zeolith vereint werden. Es ist vorstellbar, dass die Behandlungen zwecks weiterer Modifizierungen hintereinander oder aber auch gleichzeitig vorgenommen werden können. Die Verwendung eines derartig modifizierten Zeoliths ermöglicht eine gezieltere Abstimmung auf das Gärsubstrat.

Vorteilhafterweise wird als Ausgangsprodukt ein natürlicher Na-reicher Zeolith eingesetzt. Es konnte beobachtet werden, dass sich diese Zeolithart aufgrund der Morphologie, Partikelgröße und gleichförmigen bzw. regelmäßigen Hohlraumstruktur von vornherein positiv auf die Gärprozesse auswirken kann. Er zeigt eine gute Ionenaustauschkapazität, ist selektiver gegenüber spezifische Ionen, insbesondere der Ammonium-Ionen, und weist günstige Hydrationseigenschaften und Adsorptionskapazität auf. Er bietet sich daher bevorzugt als Ausgangsprodukt für den modifizierten Zeolith an.

Es ist günstig, als Zeolith kationen-aktivierten Zeolith zu verwenden. Ein solcher ist einfach und kostengünstig herzustellen und kann einen wesentlichen Beitrag zur chemisch-katalytischen Aktivität der Vergärung beisteuern, insbesondere hinsichtlich der Anfahrts- bzw. Startzeiten der Gärungsprozesse im Fermenter.

Der Erhalt eines kationen-aktivierten Zeolithen kann auf an sich bekannte Weise erfolgen.

Ein kationen-aktivierter Zeolith kann beispielsweise ein Natrium-aktivierter, bevorzugterweise ein Kalium-aktivierter Zeolith sein. Eine chemische Behandlung zum Erhalt eines Kaliumaktivierten Zeoliths kann auch beispielsweise mittels einer Kaliumchlorid-Lösung vorgenommen werden. Dadurch werden die in einem Zeolith vorhandenen Erdalkali-Ionen, insbesondere Calcium, gegen Kalium-Ionen ausgetauscht. Es kann damit ein Zeolith mit der Summenformel K₁₆(A₁₆Si₃₀O₇₂) 24 H₂O erhalten werden. Es konnte beobachtet werden, dass durch eine derartige Aktivierung die Ionenaustauschkapazität gegenüber Wasserstoff- und Ammonium-Ionen, erhöht und beschleunigt werden kann. Dies zeigt ein positive Wirkung auf die pH-Stabilität und Ammoniumtoxizität im Fermenter. Das ausgetauschte Kalium ist wiederum im Gärrückstand ein wichtiger Bestandteil für die weitere Nutzung, beispielsweise in der Rotte.

In manchen Fällen kann es gewünscht sein, als Zeolith einen Stickstoff-aktivierten Zeolith zu verwenden. Beispielsweise kann ein natürlicher Zeolith mit einer Ammonium-Lösung behandelt werden, wodurch ein Ionenaustausch der Erdalkali-Ionen durch Ammonium-Ionen erfolgt und ein Ammonium-Zeolith der allgemeinen Summenformel NH₄(Al₆Si₃₀O₇₂) 24H₂O gebildet wird. Im Fall von Gärsubstrat aus überwiegend Abfällen mit geringem Eiweißgehalt, wie beispielsweise Laub, Gräser, Baumschnitt, etc., kann der Stickstoffmangel durch Verwendung von Stickstoff-aktiviertem Zeolith ausgeglichen und damit das Nährstoffverhältnis (CNP-Verhältnis) im Gärsubstrat eingeregelt werden.

Dem Gärsubstrat kann auch ein Wasserstoff-aktivierter Zeolith beigemengt werden, wodurch der katalytische Effekt im Gärsubstrat erhöht werden kann. Es hat sich gezeigt, dass sich diese Art des Zeoliths durch eine erhöhte Brönsted-Azidität auszeichnet. Für den Erhalt eines Wasserstoff-aktivierten Zeolith kann beispielsweise von einem Stickstoff-aktivierten Zeolith ausgegangen werden, wobei er einer thermischen Behandlung bis zu 400°C unterzogen wird.

Je nach Bedarf bzw. Ausgangsbedingungen im Gärsubstrat kann sich die Verwendung eines mit zumindest einem Polymer, insbesondere einem kationenaktiven Polymer, versetzten Zeoliths als günstig erweisen. Damit liegt ein polyelektrolytischer Zeolith im Gärsubstrat vor, welcher in der Art eines Flockungsmittels reagieren kann. Dadurch kann eine Agglomeration des Feinanteils im Gärsubstrat stattfinden, was mit einer Vergrößerung der Oberfläche der Gärsubstrat-Teilchen einhergeht. Der bioaktive Abbau kann für die Methanbakterien somit erleichtert werden. Die chemische oder chemisch-physikalische Behandlung zum Versetzen des Zeoliths mit zumindest einem Polymer, kann im Zuge der Aufbereitung, z.B. Mahlung, des Zeoliths vorgenommen werden. Der Zeolith kann somit mit zumindest einem Polymer "gecoatet" werden. Beispielsweise können hiefür natürliche und bzw. oder synthetische Polymere, beispielsweise Stärke, Zellulose, Alginate und dergleichen in kationisierter Form herangezogen werden.

Gemäß einem weiteren Merkmal der Erfindung kann neben dem Zeolith als chemisch-katalytisches Additiv im Gärsubstrat auch die Biologie im Gärsubstrat mithilfe von Zeolith leistungsgesteigert werden. In diesem Fall kann als Zeolith ein mit Enzymen beladener Zeolith verwendet werden. Die Enzyme können dabei auf der Oberfläche und bzw. oder in den Hohlräumen (Makroporen) des Zeoliths sorptiv aufgenommen sein. Die Enzyme betreffen jene Enzyme bzw. Fermente, die im Zuge des anaeroben mikrobiologischen Abbauprozesses der Biomasse typischerweise gebildet werden. Zu solchen Enzymen zählen beispielsweise Zellulasen, die insbesondere bei Gärsubstrat mit höherem Anteil an Baumschnitt, hilfreich sind, da sie den Abbau der Zellulose begünstigen. Zeolithe in dieser genannten Art können die biologische Aktivität, insbesondere die Zellteilung der Bakterien, anregen und somit die Faulzeit erheblich beschleunigen.

Dies kann auch im Falle einer Verwendung von Zeolith erzielt werden, der mit Spurenelementen und/oder Wuchsstoffen und bzw. oder mit Mikroorganismen beladen ist. Ein Zusatz dieser Art von Zeolith kann einen Mangel an für die Methangasbakterien wichtigen Spurenelemente und/oder Wuchsstoffen ausgleichen. Beispielsweise kann dem Gärsubstrat mit Cobalt als Spurenelement beladener Zeolith und/oder beispielsweise mit Cystein als Wuchsstoff beladener Zeolith zugegeben werden. Unter Mikroorganismen werden verständlicherweise die Methangasbakterien verstanden, welche typischerweise am Gärungsprozess - je nach Gärungssubstrat - teilhaben. Durch Zusatz von mit Mikroorganismen beladenen Zeolithen kann eine selektive Mikroben-Addition in das Gärsubstrat auf einfache und effektive Weise erzielt werden, wodurch die biologische Aktivität angeregt und beschleunigt wird.

In diesem Zusammenhang kann beispielsweise ein Zeolith eingesetzt werden, welcher in der EP 0 937 685 A2 beschrieben ist.

Für manche Anwendungen kann die Verwendung eines mit zumindest einer komplexierenden Substanz versetzten Zeoliths von Vorteil sein. Dadurch kann beispielsweise mit Spurenelementen und/oder Wuchsstoffen versetzter Zeolith vor chemischer Zersetzung in der Gärflüssigkeit bewahrt werden und für die Methanbakterien verfügbar gehalten werden. Unter komplexierender Substanz ist bekannterweise eine chemische Substanz zu verstehen, die mit anderen chemischen Substanzen chemische Komplexe bildet. Hiefür können - je nach Bedarf - organische oder aber auch anorganische komplexierende Substanzen geeignet sein. Beispielsweise kann NTA oder andere, ähnliche Komplexbildner zur Modifizierung eines Zeoliths herangezogen werden.

Bei Bedarf, abhängig von der Art des Gärsubstrats, können die vorstehend genannten Arten der Zeolithe auch kombiniert werden, um die Anpassung an das Gärsubstrat zu verbessern und um die Gärverhältnisse noch mehr zu optimieren.

In diesem Zusammenhang ist es gemäß einem weiteren Merkmal der Erfindung vorteilhaft, wenn eine Mischung aus ein oder mehreren natürlichen und ein oder mehreren modifizierten Zeolithen verwendet wird. Es kann auch gewünscht sein, eine Mischung aus zwei verschieden modifizierten Zeolithen zu verwenden.

Gemäß einem Merkmal der Erfindung kann Zeolith in einer Korngröße von kleiner als 2, 5 mm, vorzugsweise 1 mm, verwendet werden. Dadurch kann vermieden werden, dass sich Zeolithpartikel im Fermenter absetzen und für das Gärsubstrat ungenutzt bleiben. Am bevorzugtesten ist eine Korngröße von kleiner als 100 µm, vorzugsweise von kleiner als 50 µm.

Es sollte darauf geachtet werden, dass sich die Zeolithpartikel im Gärsubstrat auf einfache Weise verteilen können, um so allen Methanbakterien zur Verfügung stehen zu können.

Eine einfache Art der Verwendung des Zeoliths ist in Pulverform oder Granulatform. Manchmal kann es auch gewünscht sein, Zeolith dem Gärsubstrat als wässrige Suspension zuzugeben, wobei die Suspension vorteilhafterweise Stabilisatoren aufweist. Selbstverständlich sind andere Formen ebenfalls denkbar, beispielsweise Zeolithe in Pellet- oder Tablettenform. Beispielsweise kann der Zeolith in gepresseter Form gegebenenfalls unter Zuhilfenahme von Bindemitteln verwendet werden. Hier ist allerdings darauf zu achten, dass sich die Zeolith-Pellets oder Zeolith-Tabletten leicht "auflösen" bzw. "zerfallen", sodass sich die einzelnen Zeolithpartikel im Gärsubstrat ungehindert fein verteilen können.

Aufgrund der vielfältigen Reaktionsfähigkeit - "Multifunktionalität" - des Zeoliths ist er besonders gut als faulzeitverkürzender und zellteilungsoptimierender Zusatz in den stark variierenden Gärsubstraten zur Methangasgewinnung geeignet.

Gemäß einem weiteren Aspekt der Erfindung kann modifizierter Zeolith zur Gewinnung von Methangas als Zusatz eines Gärsubstrats in einer Menge von etwa 0,1% bis zu etwa 10% bezogen auf die organische Substanz des Gärsubstrats verwendet werden.

Die Erfindung wird im folgenden anhand von Beispielen näher erläutert.

### Beispiel 1:

In einem Laborexperiment wurden Substratmischungen aus Schweinegülle und Maissilage unter Zusatz von Faulbakterien aus einer Biogasanlage bei 35°C vergoren. Die entstehende Menge Biogas wurde in einer Waschflasche mit 100 mL 2 molare NaOH-Lösung durch Absorption vom Kohlendioxid befreit und das verbleibende Gasvolumen wurde volumetrisch durch Verdrängung einer Sperrflüssigkeit, bestehend aus wässriger Schwefelsäure in der Konzentration 10 g/Liter Schwefelsäure, gemessen. Das gemessene Gasvolumen wurde so vor der Messung von Kohlendioxid, Schwefelwasserstoff und Ammoniak befreit und besteht daher zu über 98% aus Methan.

Es wurden zeitgleich drei Versuche mit je 2 Parallelansätzen durchgeführt, um die unterschiedliche Wirkung von natürlichem Zeolith, einfach modifiziertem und doppelt modifiziertem Zeolith auf die Gasbildungsrate zu untersuchen. Die Substratmischungen bestanden aus 65 g Schweinegülle und 85 g Maissilage, zugesetzt 21 wurden je 400 mL Faulschlamm und 5% Zeolith, bezogen auf die organische Trockenmasse. In Ansatz 1 und 2 wurde natürlicher Zeolith verwendet, in Ansatz 3 und 4 wurde Cobalt-aktivierter Zeolith, in Ansatz 5 und 6 wurde Zeolith verwendet, der Cobalt- und Eisen-aktiviert wurde.

Die entstehenden Volumina an Methan wurden während eines Zeitraumes von 31 Tagen gemessen und aufsummiert. Die nachstehende Tabelle 1 gibt einen Überblick über die Gasbildung während dieser Zeit.

**Tabelle 1: Kumulative Methanbildung während der 31-tägigen Vergärung einer Substratmischung aus Schweinegülle und Maissilage mit Zusatz von verschiedenen Zeolithen.**

| ***Ansätze*** | ***Zeolith-Art*** | ***Kumulative Methanbildung (NL*/*kg oTS)*** | | | | | | | |
|---|---|---|---|---|---|---|---|---|---|
| | | 0 Tage | MW | 10 Tage | MW | 20 Tage | MW | 31 Tage | MW |
| 1 | Nat. Zeolith | 0 | 0 | 34,8 | 37,1 | 53,2 | 55,9 | 65,6 | 63,3 |
| 2 | Nat. Zeolith | 0 | | 39,4 | | 58,6 | | 61,1 | |
| 3 | Co-Zeolith | 0 | 0 | 40,28 | 39,3 | 54,8 | 55,0 | 72,3 | 71, 5 |
| 4 | Co-Zeolith | 0 | | 38,4 | | 55,2 | | 70,6 | |
| 5 | Co-Fe-Zeolith | 0 | 0 | 56,1 | 52,3 | 76,4 | 80,0 | 108,0 | 99,0 |
| 6 | Co-Fe-Zeolith | 0 | | 48,6 | | 83,5 | | 89,7 | |

| | | | | | | | | | |
|---|---|---|---|---|---|---|---|---|---|
| Anmerkung: NL = Normliter, MW = Mittelwert, oTS = organische Trockensubstanz, Nat. Zeolith = Natürlicher Zeolith, Co- Zeolith = Cobalt-aktivierter Zeolith, Co-Fe- Zeolith = Cobalt- und Eisen- zweifach modifizierter Zeolith. | | | | | | | | | |

Die Ergebnisse in Tabelle 1 zeigen deutlich, dass
1.ein signifikanter Unterschied in der Wirkung auf die Gasbildungsrate zwischen nichtmodifiziertem Zeolith, einfach und zweifach modifiziertem Zeolith besteht;
2.die Gasbildungsrate bei Zugabe von Co-Fe- Zeolith um ca. 30% höher liegt als bei Co-aktiviertem Zeolith.

## Patentansprüche

1. Verwendung von aktiviertem Zeolith zur Gewinnung von Methangas, wobei der Zeolith als prozessstabilisierender, den Abbau von Gärsubstrat aktivierender und in Biogas vorhandenes ammoniak- und schwefelwasserstoffsenkender Zusatz sowie als zellteilungsoptimierender und faulzeitverkürzender Zusatz eines Gärsubstrats in einer Menge von etwa 0,1% bis zu etwa 10% bezogen auf die organische Substanz des Gärsubstrats verwendet wird, **dadurch gekennzeichnet, dass** als Zeolith ein Zeolith zugesetzt wird, der Cobalt- und Eisen aktiviert wurde.

2. Verwendung nach Anspruch 1, **dadurch gekennzeichnet, dass** als Ausgangsprodukt des aktivierten Zeoliths natürlicher Zeolith ausgewählt aus Klinoptilolith, Chabasit, Phillipsit, Analcim oder ähnliche, sowie Mischungen hievon, verwendet wird.

3. Verwendung nach einem der Ansprüche 1 oder 2, **dadurch gekennzeichnet, dass** kationen-aktivierter Zeolith verwendet wird.

4. Verwendung nach Anspruch 3, **dadurch gekennzeichnet, dass** Kalium-aktivierter Zeolith verwendet wird.

5. Verwendung nach Anspruch 3 oder 4, **dadurch gekennzeichnet, dass** Stickstoff-aktivierter Zeolith verwendet wird.

6. Verwendung nach einem der Ansprüche 3 bis 5, **dadurch gekennzeichnet, dass** Wasserstoff-aktivierter Zeolith verwendet wird.

7. Verwendung nach einem der Ansprüche 1 bis 6, **dadurch gekennzeichnet, dass** mit zumindest einem Polymer, insbesondere kationenaktiven Polymer, versetzter Zeolith verwendet wird.

8. Verwendung nach einem der Ansprüche 1 bis 7, **dadurch gekennzeichnet, dass** mit Enzymen beladener Zeolith verwendet wird.

9. Verwendung nach einem der Ansprüche 1 bis 8, **dadurch gekennzeichnet, dass** mit Spurenelementen und/oder Wuchsstoffen beladener Zeolith verwendet wird.

10. Verwendung nach einem der Ansprüche 1 bis 9, **dadurch gekennzeichnet, dass** mit Mikroorganismen beladener Zeolith verwendet wird.

11. Verwendung nach einem der Ansprüche 1 bis 10, **dadurch gekennzeichnet, dass** mit komplexierenden Substanzen beladener Zeolith verwendet wird.

12. Verwendung nach einem der Ansprüche 1 bis 11, **dadurch gekennzeichnet, dass** eine Mischung aus modifiziertem Zeolith und natürlichem Zeolith verwendet wird.

13. Verwendung nach einem der Ansprüche 1 bis 12, **dadurch gekennzeichnet, dass** eine Mischung aus zwei verschieden modifierten Zeolithen verwendet wird.

14. Verwendung nach einem der Ansprüche 1 bis 13, **dadurch gekennzeichnet, dass** Zeolith in einer Korngröße von kleiner als 2,5 mm, vorzugsweise kleiner als 1 mm, verwendet wird.

15. Verwendung nach einem der Ansprüche 1 bis 14, **dadurch gekennzeichnet, dass** Zeolith in wässriger Suspension zugesetzt wird.

16. Verwendung nach einem der Ansprüche 1 bis 15, **dadurch gekennzeichnet, dass** Zeolith in Pellet- oder Tablettenform verwendet wird.

## Claims

1. Use of activated zeolite for generating methane gas, whereby the zeolite is used as a process-stabilising additive which activates the breakdown of the fermentation substrate and reduces the ammonia and hydrogen sulphide present in the biogas, and as an additive of a fermentation substrate which optimises cell distribution and shortens fermentation time used in a quantity of approximately 0.1% to approximately 10% by reference to the organic substance of the fermentation substrate used, **characterised in that** a cobalt- and iron activated zeolite is added as the zeolite.

2. Use as claimed in claim 1, **characterised in that** natural zeolite selected from klinoptilolite, chabasite, phillipsite, analcim or similar and mixtures thereof is used as an initial product for the activated zeolite.

3. Use as claimed in one of claims 1 or 2, **characterised in that** cation-activated zeolite is used.

4. Use as claimed in claim 3, **characterised in that** potassium-activated zeolite is used.

5. Use as claimed in claim 3 or 4, **characterised in that** nitrogen-activated zeolite is used.

6. Use as claimed in one of claims 3 to 5, **characterised in that** hydrogen-activated zeolite is used.

7. Use as claimed in one of claims 1 to 6, **characterised in that** zeolite displaced with a polymer, in particular cation-activated polymer, is used.

8. Use as claimed in one of claims 1 to 7, **characterised in that** zeolite entraining enzymes is used.

9. Use as claimed in one of claims 1 to 8, **characterised in that** zeolite entraining trace elements and/or growth substances is used.

10. Use as claimed in one of claims 1 to 9, **characterised in that** zeolite entraining microorganisms is used.

11. Use as claimed in one of claims 1 to 10, **characterised in that** zeolite entraining complexing agents is used.

12. Use as claimed in one of claims 1 to 11, **characterised in that** a mixture of modified zeolite and natural zeolite is used.

13. Use as claimed in one of claims 1 to 12, **characterised in that** a mixture of two different modified zeolites is used.

14. Use as claimed in one of claims 1 to 13, **characterised in that** zeolite with a grain size of less than 2.5 mm, preferably less than 1 mm, is used.

15. Use as claimed in one of claims 1 to 14, **characterised in that** zeolite is added in an aqueous suspension.

16. Use as claimed in one of claims 1 to 15, **characterised in that** zeolite in the form of pellets or tablets is used.

## Revendications

1. Utilisation de zéolite activée pour l'obtention de gaz méthane où la zéolite est utilisée en tant qu'additif stabilisant le procédé, activant la décomposition d'un substrat nutritif et abaissant les teneurs en ammoniaque et en eau sulfhydrique, ainsi qu'optimisant la répartition des cellules et raccourcissant le temps de fermentation d'un substrat alimentaire, en une quantité d'environ 0,1% à environ 10% en se rapportant à la substance organique du substrat de fermentation, **caractérisée en ce qu'**on utilise, en tant que zéolite une zéolite activée par du cobalt et du fer.

2. Utilisation selon la revendication 1, **caractérisée en ce qu'**en tant que matière première de la zéolite activée, on utilise une zéolite naturelle choisie parmi la klinoptilolithe, la Chabasite, la Phillipsite, l'Analcim ou analogues, ainsi que des mélanges de celles-ci.

3. Utilisation selon l'une quelconque des revendications 1 ou 2, **caractérisée en ce qu'**on utilise une zéolite activée par les cations.

4. Utilisation selon la revendication 3, **caractérisée en ce qu'**on utilise une zéolite activée par du potassium.

5. Utilisation selon la revendication 3 ou 4, **caractérisée en ce qu'**on utilise une zéolite activée par de l'azote.

6. Utilisation selon l'une quelconque des revendications 3 à 5, **caractérisée en ce qu'**on utilise une zéolite activée par de l'hydrogène.

7. Utilisation selon l'une quelconque des revendications 1 à 6, **caractérisée en ce qu'**on utilise une zéolite mélangée à au moins un polymère, en particulier, un polymère cationiquement actif.

8. Utilisation selon l'une quelconque des revendications 1 à 7, **caractérisée en ce que** l'on utilise une zéolite chargée d'enzymes.

9. Utilisation selon l'une quelconque des revendications 1 à 8, **caractérisée en ce que** l'on utilise une zéolite chargée d'oligo-éléments et/ou de substances de croissance.

10. Utilisation selon l'une quelconque des revendications 1 à 9, **caractérisée en ce qu'**on utilise une zéolite chargée de microorganismes.

11. Utilisation selon l'une quelconque des revendications 1 à 10, **caractérisée en ce que** l'on utilise une zéolite chargée de substances complexantes.

12. Utilisation selon l'une quelconque des revendications 1 à 11, **caractérisée en ce qu'**on utilise un mélange de zéolite modifiée et de zéolite naturelle.

13. Utilisation selon l'une quelconque des revendications 1 à 12, **caractérisée en ce qu'**on utilise un mélange de deux zéolites modifiées différentes.

14. Utilisation selon l'une quelconque des revendications 1 à 13, **caractérisée en ce que** l'on utilise une zéolite à une granulométrie de moins de 2,5 mm, avantageusement de moins de 1 mm.

15. Utilisation selon l'une quelconque des revendications 1 à 14, **caractérisée en ce qu'**on utilise une zéolite dans une suspension aqueuse.

16. Utilisation selon l'une quelconque des revendications 1 à 15, **caractérisée en ce qu'**on utilise une zéolite sous forme de boulette ou de comprimés.
